# EUROPEAN PATENT SPECIFICATION

(11) **EP 4 440 689 B1**
(45) Date of publication and mention of the grant of the patent: **20.08.2025**
(21) Application number: 22822495.2
(22) Date of filing: 28.11.2022
(51) Int. Cl.: A61N 1/375, A61N 1/39, A61N 1/365, A61N 1/37, A61N 1/372

(54) **IMPLANTABLE MEDICAL DEVICE FOR PROVIDING A DIAGNOSTIC AND/OR THERAPEUTIC CARDIAC FUNCTION WITHIN A PATIENT**
IMPLANTIERBARE MEDIZINISCHE VORRICHTUNG ZUR BEREITSTELLUNG EINER DIAGNOSTISCHEN UND/ODER THERAPEUTISCHEN HERZFUNKTION IN EINEM PATIENTEN
DISPOSITIF MÉDICAL IMPLANTABLE POUR LA FOURNITURE D'UN DIAGNOSTIC ET/OU D'UNE FONCTION CARDIAQUE THÉRAPEUTIQUE CHEZ UN PATIENT

(30) Priority: 01.12.2021 EP 21211619
(43) Date of publication of application: 09.10.2024
(73) Proprietor: BIOTRONIK SE & Co. KG, 12359 Berlin (DE)
(72) Inventor: DOERR, Thomas, 12437 Berlin (DE); WEISS, Ingo, 12435 Berlin (DE)
(74) Representative: Biotronik Corporate Services SE
(86) International application number: PCT/EP2022/083433
(87) International publication number: WO 2023/099385

(56) References cited:
- US-A1- 2018 043 158
- US-A1- 2019 111 270
- US-A1- 2019 329 045
- US-A1- 2020 215 340
- US-B1- 9 168 380
- US-B2- 9 675 811

## Description

The instant invention generally relates to an implantable medical device for providing a diagnostic and/or therapeutic cardiac function within a patient, to an implantable therapy system for providing a diagnostic and/or therapeutic cardiac function within a patient.

Generally, an implantable medical device comprises a sensing arrangement for sensing electrocardiogram signals, a communication circuitry for data communication with a device external to the patient, and a processing circuitry for processing sensed electrocardiogram signals.

According to an embodiment of the present invention, the communication circuitry for data communication is configured to communicate with a device external to the patient.

An implantable medical device of the type concerned herein for example may be an implantable cardioverter defibrillator (ICD), which in particular may be configured for a non-transvenous implantation external to the patient's heart.

Within an implantable therapy system, multiple implantable medical devices may be implanted in a patient, for example an implantable medical device in the shape of an implantable cardioverter defibrillator together with a pacemaker device, such as a leadless pacemaker. Devices of the implantable therapy system herein shall communicate with an external device, for example within a home monitoring system, such that information concerning devices of the implantable therapy system is communicated to the external device and, via the external device, may be relayed e.g. to a home monitoring service center for assessment e.g. by a physician.

Within an implantable therapy system, some devices may be implanted deeper in a patient than others. In addition, some devices, such as for example a leadless pacemaker device, may be small in construction and hence may comprise only a comparatively small battery comprising a limited energy storage capacity, such that those devices shall operate in a particularly energy-efficient manner.

In order to allow for a communication between devices of an implantable therapy system, approaches exist for establishing an intra-body communication network. Intra-body communication techniques however require the implantable medical devices to interact in a defined manner, posing rather strict compatibility restrictions on the implantable medical devices.

In other approaches, a communication with implantable medical devices implanted deep in a patient may involve low frequency communication techniques, for example employing a coil telemetry communication. This however requires the use of a reading device which must be approached externally to the patient in order to establish a data transmission, making a communication potentially unreliable and cumbersome for a user.

EP 2 327 609 B1 describes an acoustic communication link in between implanted medical devices for exchanging information in between the implanted medical devices. The acoustic communication link is established to permit wireless communication between the implanted medical devices, wherein transmission parameters may be adapted, such as a sensitivity and a carrier frequency, in order to improve an existing communication link.

US 2010/0022836 A1 discloses multidirectional transmitters for in-body devices, such as implantable devices.

US 9 168 380 B1 discloses a medical device system including an intracardiac pacemaker that is configured to receive by an implantable medical device sensing module a first cardiac signal using a first pair of electrodes implanted outside the cardiovascular system and identify a P-wave from the first cardiac signal. The system transmits a wireless trigger signal to the intracardiac pacemaker in response to identifying the P-wave. The intracardiac pacemaker delivers a pacing therapy in response to the trigger signal.

US 9 675 811 B2 discloses a cardiac rhythm management system including a first implantable device such as a defibrillator and a second implantable device such as a leadless cardiac pacemaker. A programmer is configured to receive and display heart data emanating from the implantable defibrillator and from the leadless cardiac pacemaker. The heart data emanating from the leadless cardiac pacemaker is displayed in temporal alignment with the heart data emanating from the implantable defibrillator.

US 2020 215 340 A1 discloses an implantable system including an atrial leadless pacemaker (aLP) and a ventricular leadless pacemaker (vLP), and methods for use therewith, that are configured or used to terminate a pacemaker mediated tachycardia (PMT). One of the aLP or the vLP detects a PMT and informs the other one. The aLP initiates a PMT PA interval that is shorter than a PA interval that the aLP would otherwise use for atrial pacing if a PMT was not detected. The vLP initiates a PMT PV interval that is longer than the PMT PA interval. If an intrinsic atrial or ventricular event is detected before PMT PA interval or the PMT PV interval expires, then these intervals will be terminated, otherwise an atrial chamber will be paced if the PMT PA interval expires, and/or a ventricular chamber will be paced if the PMT PV interval expires. This should have the effect of terminating the PMT.

US 2018 004 3158 A1 discloses systems and methods for monitoring patients with cardiac diseases. A system may include an accelerometer sensor for sensing an epicardial or endocardial acceleration (EA) signal. The accelerometer may be included in a leadless or a lead-based medical device. The system may additionally include a companion device in communication with the medical device via a wireless communication link. The companion device may sense a cardiac signal such as a heart sound signal, and may use the cardiac signal and the EA signal transmitted from the medical device to generate a diastolic function indicator indicating the diastolic function of the heart. The system may provide the diastolic function indicator to a user or a process, or to detect worsening of heart failure based on the diastolic function indicator.

US 2019 011 1270 A1 deals with pacing of cardiac tissue, and more particularly with adjusting delivery of His bundle or bundle branch pacing in a cardiac pacing system to achieve synchronized ventricular activation. Bundle pacing may be delivered in response to determining whether the QRS parameter or activation interval is greater than a threshold. A set of AV delays may be generated, and an optimal AV delay may be selected from the stored set of AV delays. His-bundle or bundle-branch pacing may be selectively delivered based on RV or LV activation time. Pacing may also be adjusted based on dyssynchrony detected or the type of bundle branch block pattern detected.

US 2019 329 045 A1 discloses an implantable medical device system including an extracardiac sensing device and an intracardiac pacemaker. The sensing device senses a P-wave attendant to an atrial depolarization of the heart via housing-based electrodes carried by the sensing device when the sensing device is implanted outside the cardiovascular system and sends a trigger signal to the intracardiac pacemaker in response to sensing the P-wave. The intracardiac pacemaker detects the trigger signal and schedules a ventricular pacing pulse in response to the detected trigger signal.

It is an object of the instant invention to provide an implantable medical device and an implantable therapy system which allow for an easy and reliable monitoring and information transmission concerning a device which may be implanted deep in a patient.

This object is achieved by means of an implantable medical device comprising the features of claim 1. Aspects, embodiments and examples disclosed herein which do not fall within the scope of the appended claims do not form part of the invention, and are merely provided for illustrative purposes.

In one aspect, an implantable medical device for providing a diagnostic and/or therapeutic cardiac function within a patient, comprises: a sensing arrangement for sensing electrocardiogram signals; a communication circuitry for data communication with a device external to the patient; and a processing circuitry for processing sensed electrocardiogram signals, wherein the processing circuitry is configured to identify, based on the sensed electrocardiogram signals, a stimulation event caused by an implantable pacemaker device and to cause a transmission of information concerning said identification of a stimulation event to the device external to the patient.

The implantable medical device may in particular be an implantable non-transvenous defibrillator device configured to emit a shock pulse for achieving a defibrillation. An implantable non-transvenous defibrillator device, also denoted as non-transvenous implantable cardioverter defibrillator (in short non-transvenous ICD), may serve for monitoring and treating potentially life-threatening arrhythmias of a patient's heart. The implantable non-transvenous defibrillator device is configured for non-transvenous implantation, that is an implantation such that no electrode leads transvenously are implanted within the heart of a (human or animal) patient. The implantable non-transvenous defibrillator device hence is to be implanted in a patient such that a generator and an electrode arrangement, for example a shock electrode placed on a lead connected to the generator, are implanted extracardially and do not reach into the heart of the patient, that is into the right or left ventricle or the right or left atrium.

In one embodiment, the implantable non-transvenous defibrillator device comprises a lead to be implanted extracardially and a shock electrode arranged on the lead. The shock electrode herein, in an implanted state of the defibrillator device, is placed outside of the heart of the patient, for example in the region of the sternum of the patient, such that a shock pulse for achieving a defibrillation is generated outside of the heart. Generally, the implantable non-transvenous defibrillator device does not comprise any portions which extend transvenously into the heart, but the defibrillator device is configured to achieve a sensing and emission of signals outside of the heart.

The implantable medical device comprises a sensing arrangement for sensing electrocardiogram signals. The sensing arrangement may for example comprise one or multiple pairs of electrode poles via which electrocardiogram signals may be sensed and forwarded to processing circuitry operatively connected to the sensing arrangement.

The processing circuitry is configured to process sensed electrocardiogram signals, wherein the processing circuitry is configured to identify, within the sensed electrocardiogram signals, stimulation events caused by an implantable pacemaker device. The implantable medical device hence is programmed to derive, from sensed electrocardiogram signals, information relating to another implantable device, namely an implantable pacemaker device, which is separate and independent from the implantable medical device. The implantable pacemaker device in particular may be a leadless pacemaker device which is directly implanted into the heart of the patient, for example into the right or left ventricle, and which does not comprise leads carrying electrodes.

In that the implantable medical device, which may in particular be implanted extracardially, identifies stimulation events as caused by the implantable pacemaker device within sensed electrocardiogram signals and communicates information concerning the identification of stimulation events to an external device, the implantable medical device may establish a monitoring function for the implantable pacemaker device. Information relating to the operation of the implantable medical device hence may be monitored by the implantable medical device and may be communicated by the implantable medical device to an external device. In this way data concerning the operation of the implantable pacemaker device is obtained using the implantable medical device and is communicated to the external device by means of the implantable medical device, hence making a direct data communication in between the implantable pacemaker device and the external device dispensable.

In principle, hence, the implantable pacemaker device does not need to establish a communication to the external device, such that the implantable pacemaker device may be operated in a particularly energy-efficient manner.

A monitoring of the operation of the implantable pacemaker device herein may reliably be established even if the implantable pacemaker device is implanted deep in the patient, hence making a direct data communication of the implantable pacemaker device to an external device potentially difficult. In that the monitoring of the implantable pacemaker device is carried out by the implantable medical device based on the sensing of electrocardiogram signals, no data transmission from the implantable pacemaker device to an external device is required to establish a monitoring, such that a deep implantation state of the implantable pacemaker device does not impact data communication.

The implantable medical device is configured to identify stimulation events caused by the implantable pacemaker device based on sensed electrocardiogram signals. Within regular operation the implantable medical device records electrocardiogram signals and processes sensed electrocardiogram signals, wherein based on the processing stimulation events as caused by the implantable pacemaker device are identified and information relating to the stimulation events is derived and is transmitted to the external device. As the identification of the stimulation events does not require a direct communication in between the implantable pacemaker device and the implantable medical device, requirements for a compatibility of the devices are negligible. A monitoring of an implantable pacemaker device hence may be established for example even in scenarios in which the implantable pacemaker device and the implantable medical device originate from different manufacturers.

In one embodiment, the processing circuitry is configured to store a portion of the sensed electrocardiogram signals dependent on said identification of a stimulation event and to transmit a stored portion of the sensed electrocardiogram signals to the device external to the patient. If a stimulation event is identified, signal portions relating to the stimulation event may be stored and may be communicated to the external device, such that signal portions indicative of the stimulation events are forwarded to the external device for further processing.

In one embodiment, the processing circuitry comprises a circular buffer for circularly storing sensed electrocardiogram signals. The circular buffer, also denoted as ring memory, may for example comprise a storage capacity for storing a signal portion of a specified time length, for example in a range between 10 seconds and 5 minutes, in particular in between 20 seconds and 1 minute. The circular buffer herein overwrites circularly its memory contents, such that the circular buffer at any time contains a signal portion of a defined prior time span.

In case a stimulation event caused by the implantable pacemaker device is identified, the processing circuitry may cause a transmission of a data message containing a signal portion prior to the stimulation event, a signal portion containing the stimulation event and/or a signal portion after the stimulation event. Resulting from the identification of a stimulation event, hence, a signal portion is extracted from the circular buffer and is forwarded to the external device, such that electrocardiogram signals indicative of the stimulation event are transmitted to the external device.

Alternatively or in addition, the processing circuitry may be configured to statistically analyze stimulation events as identified based on the electrocardiogram signals. Statistical information concerning identified stimulation events may then be derived by the processing circuitry and may be communicated to the external device.

In one embodiment, the processing circuitry is configured to store programming information indicative of a programmed stimulation function of the implantable pacemaker device, wherein the processing circuitry is configured to monitor the programmed stimulation function based on the identification of a stimulation event. The implantable medical device hence may comprise information relating to the operation of the implantable pacemaker device. The processing circuitry of the implantable medical device is programmed such that it is enabled to assess how the implantable pacemaker device should function and to evaluate whether the implantable pacemaker device functions correctly according to its programmed stimulation function. If it is found that the implantable pacemaker device in its operation deviates from the programmed stimulation function, information in this respect, including for example a warning message, may be sent to the external device.

For example, the processing circuitry may be configured to identify, based on the sensed electrocardiogram signals, a requirement for a stimulation event and to monitor, based on the sensed electrocardiogram signals, whether the implantable pacemaker device causes a stimulation event in relation to an identification of a requirement for a stimulation event. If the processing circuitry identifies, based on the sensed electrocardiogram signals, that in a particular situation the implantable pacemaker device should trigger a stimulation event based on a programmed stimulation function of the implantable pacemaker device, the processing circuitry monitors the sensed electrocardiogram signals whether the stimulation event in fact occurs. If the stimulation event occurs, the processing circuitry of the implantable medical device may identify that the implantable pacemaker device functions according to its prescribed programming. If the stimulation event does not occur, the processing circuitry may identify that the implantable pacemaker device does not operate according to its programmed stimulation function and may accordingly send information to the external device.

In an example, the processing circuitry may be configured to identify an anti-tachycardia stimulation event caused by the implantable pacemaker device and to cause a transmission of information concerning the anti-tachycardia stimulation event to the external device. The programmed stimulation function of the implantable pacemaker device may for example relate to an anti-tachycardia stimulation (ATP) in order to provide a therapy for a ventricular tachycardia. If the implantable medical device identifies an anti-tachycardia stimulation event, information relating to the anti-tachycardia stimulation event may be communicated to the external device.

In another example, the processing circuitry may be configured to identify a requirement for an anti-bradycardia stimulation and to monitor whether the implantable pacemaker device causes an anti-bradycardia stimulation event in relation to an identification of a requirement for an anti-bradycardia stimulation. The implantable pacemaker device hence is programmed to provide for a therapy in case a bradycardia occurs. If the implantable medical device identifies a bradycardia and hence assesses that an anti-bradycardia stimulation event should be caused by the implantable pacemaker device, but in the further monitoring detects that no stimulation by the implantable pacemaker device takes place, this may indicate an incorrect functioning of the implantable pacemaker device, such that information in this respect, for example including a warning message, may be transmitted to the external device.

The processing circuitry of the implantable medical device generally may be configured to identify a stimulation event caused by the implantable pacemaker device and to monitor a subsequent stimulated cardiac response, for example in a predefined time window following the identified stimulation event. In this way the processing circuitry may monitor a cardiac response and may classify a stimulation, based on the cardiac response, as effective or ineffective. Information in this respect may be transmitted to the external device.

In one embodiment, the processing circuitry is configured to trigger a data communication message to the device external to the patient based on the identification of a stimulation event. Hence, in an event-based approach a data communication may be initiated for example immediately upon identifying a stimulation event or based on a monitoring event indicating a correct or incorrect stimulation operation by the implantable pacemaker device. Alternatively or in addition, information concerning said identification of a stimulation event may be included in a data message which is transmitted by the implantable medical device according to a predefined, regular communication scheme. The implantable medical device may for example be configured to regularly send data messages to the external device. In such regular messages also information concerning the implantable pacemaker device may be included.

In an embodiment, the implantable pacemaker device is configured to deliver at least one specific stimulation pattern to indicate at least one device condition. The processing circuitry of the implantable medical device is configured to sense and detect the specific stimulation pattern and forwards the information on the device state to the device external to the patient or to an external server. According to an embodiment, the specific pulse pattern comprises a pulse sequence which is unconventional for therapeutic cardiac pacing, as for instance a predetermined number of pulses at 70 bpm. In an embodiment, the specific pulse pattern comprises a repetition of the pulse sequence a predetermined number of times, e.g. 3 or 5 times.

According to an embodiment, the device state may indicate an ERI (Elective replacement indication) status, calling for a battery change of the implantable pacemaker. In general, the status can be any other status which is crucial to report to an external user in order to trigger an external action.

In an embodiment, the processing circuitry of the implantable medical device is configured to determine a mean stimulation rate of successive stimulation events caused by the implantable pacemaker device. In one example, the processing circuitry is configured to determine the mean stimulation rate by calculating a moving average. An example time window for ECG measurements for determining a moving average could be e.g. 10-50s, in particular 30s.

Moreover, according to an embodiment, the processing circuitry is configured to track changes of the mean stimulation rate. For instance, in case the processing circuitry identifies a reduction of mean stimulation rate by 10% or 12% of the original mean stimulation rate. A reduction of the stimulation rate can be automatically performed by the implantable pacemaker device when a low battery state is entered (e.g. ERI state), in order to save energy. By the said embodiment, the implantable medical device is enabled to detect that state change of the implantable pacemaker device, and forward the information of the state change to the device external to the patient or to an external server.

An implantable therapy system comprises, in one embodiment, an implantable pacemaker device for emitting a cardiac stimulation signal for achieving an intracardiac pacing, and an implantable medical device of the type described above. The implantable pacemaker device may for example be a leadless pacemaker device which is to be implanted directly into the heart, for example the right or left ventricle or the right or left atrium. The pacemaker device may for example provide for a therapy in case a bradycardia is detected in order to pace the patient's heart and to in this way pace the heart rate to a desired, normal heart rate. Alternatively or in addition, the pacemaker device may provide for a therapy in case a tachycardia is detected in order to reset the heart rate to a desired normal rate.

In another aspect, a method for operating an implantable medical device for providing a diagnostic and/or therapeutic cardiac function within a patient comprises: sensing, using a sensing arrangement of the implantable medical device, electrocardiogram signals; processing, using a processing circuitry of the implantable medical device, sensed electrocardiogram signals, wherein the processing includes: identifying, based on the sensed electrocardiogram signals, a stimulation event caused by an implantable pacemaker device and causing a transmission, using a communication circuitry of the implantable medical device, of information concerning an identification of a stimulation event to a device external to the patient.

The advantages and advantageous embodiments described above for the implantable medical device and the implantable therapy system equally apply also to the method, such that it shall be referred to the above in this respect.

The idea of the invention shall subsequently be described in more detail with reference to the embodiments as shown in the drawings. Herein:
- Fig. 1: shows a schematic drawing of a therapy system of medical devices implanted in a patient; and
- Fig. 2: shows a schematic drawing of an implantable medical device together with an implantable pacemaker device.

Subsequently, embodiments of the invention shall be described in detail with reference to the drawings. In the drawings, like reference numerals designate like structural elements.

It is to be noted that the embodiments are not limiting for the invention, but merely represent illustrative examples.

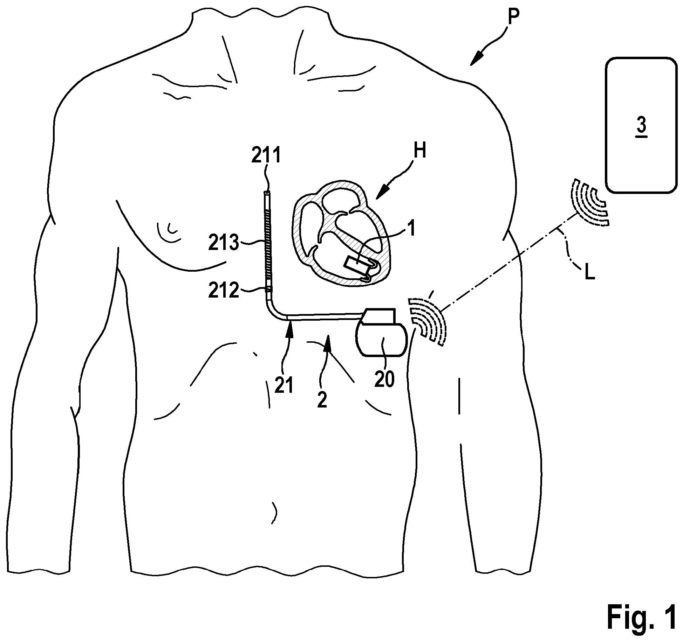

Referring to Fig. 1, in a setup of a therapy system an implantable leadless pacemaker device 1 is implanted in the heart H of a (human or animal) patient P. The leadless pacemaker device 1 is implanted directly into the heart H, for example in the right ventricle RV, in order to couple to tissue within the heart H and to in this way sense cardiac signals within the heart and emit stimulation signals into cardiac tissue.

In addition, an implantable medical device 2 in the shape of a non-transvenous implantable defibrillator device is implanted such that the implantable medical device 2 is completely external to the heart H, the non-transvenous implantable medical device 2 comprising a generator 20 encapsulated within a housing, and a lead 21 connected to the generator 20 and carrying sensing electrodes 211, 212 as well as a shock electrode 213 in the shape of a coil formed on a distal portion of the lead 21. The sensing electrodes 211, 212, for example formed as ring electrodes on either side of the shock electrode 213, serve to sense cardiac signals for processing within the generator 20 of the implantable medical device 2, such that based on sensed signals e.g. a tachycardia may be identified and a shock pulse may be generated for providing for an anti-tachycardia therapy.

In a setup as shown in Fig. 1, the pacemaker device 1 implanted within the heart H and the implantable medical device 2 implanted outside of the heart H each comprise sensing circuitry, such that based on a processing of sensed signals in the respective device 1, 2 a therapeutic action may be initiated. Each device 1, 2 herein functions, in principle, independent of the other device 2, 1, such that by means of the pacemaker device 1 implanted in the heart H stimulation signals e.g. for overcoming a bradycardia may be generated, whereas by means of the defibrillator device 2 a shock pulse may be emitted for resetting e.g. a tachycardic heart rate to a normal heart rate.

In a setup as shown in Fig. 1, the implantable pacemaker device 1 is implanted in the heart H and hence rather deep within the patient P. The implantable medical device 2, in contrast, with its generator 20 may for example be implanted subcutaneously, the lead 21 extending from the generator 20 such that it extends outside of the heart H and hence does not reach transvenously into the heart H. The implantable medical device 2 is configured to establish a communication link L to an external device 3 such that a data communication in between the implantable medical device 2 and the external device 3 may take place.

The external device 3 may for example be part of a home monitoring system. The external device 3 for example resides at the site, e.g. at the home, of the patient P and beneficially is in connection with a home monitoring service center, such that data may be relayed to and from the home monitoring center by means of the external device 3.

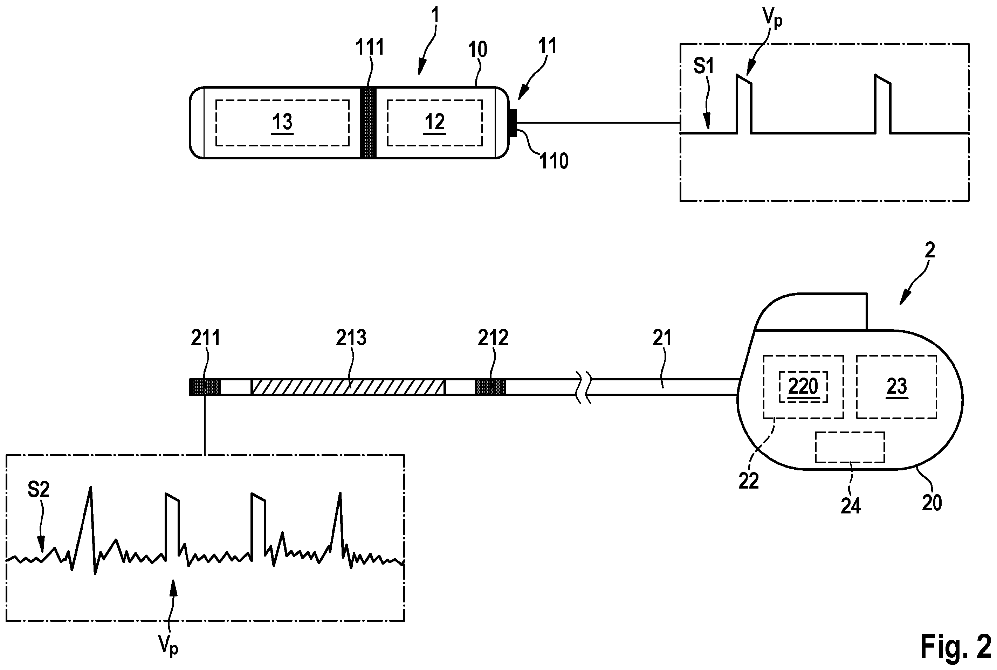

Referring now to Fig. 2, the pacemaker device 1 comprises a housing 10 on which an electrode arrangement 11 having electrode poles 110, 111 is arranged. The electrode poles 110, 111 form a pair of electrodes by means of which stimulation pulses may be injected into tissue and sense signals may be received from tissue to provide for a stimulation and sensing at the implantation site of the implantable pacemaker device 1. The implantable pacemaker device 1 furthermore comprises control circuitry 12 and a battery 13 providing for an energy storage, both the control circuitry 12 and the battery 13 being encapsulated in the housing 10.

The non-transvenous implantable medical device 2, within the generator 20, comprises processing circuitry 22, a battery 23 providing for an energy storage, and communication circuitry 24.

The processing circuitry 22 serves to process signals received via the sensing arrangement of the sensing electrodes 211, 212 and to trigger a stimulation action by emitting a shock pulse via the shock electrode 213 in case e.g. a tachycardia is detected.

By means of the communication circuitry 24 a communication link L (see Fig. 1) to the external device 3 is established, the communication circuitry 24 being configured for example to establish a communication according to the MICS protocol, the ISM protocol, the BLE protocol, the Zigbee protocol, or according to another communication scheme.

The pacemaker device 1 and the non-transvenous implantable medical device 2, in the shown embodiment, are not functioning in immediate connection with each other. That is, the pacemaker device 1 and the implantable medical device 2 are not in data connection with each other, such that no control signals are exchanged in between the pacemaker device 1 and the implantable medical device 2.

Rather, the pacemaker device 1 functions, for example, without knowledge about the presence of the implantable medical device 2 by sensing electrocardiogram signals within the heart H and by emitting stimulation signals S1 based on a processing of sensed signals. The pacemaker device 1, for example, may be configured to detect a bradycardia, and to emit stimulation pulses to cause stimulation events Vp in case a bradycardia is detected.

In the example of Figs. 1 and 2, the pacemaker device 1 does not directly communicate with the external device 3. In order to nevertheless provide for a monitoring of the operation of the pacemaker device 1, the implantable medical device 2 is configured to identify, within sensed electrocardiogram signals S2, stimulation events Vp caused by the pacemaker device 1.

For example, the sensed electrocardiogram signals S2 may be processed within the processing circuitry 22 of the generator 20 of the implantable medical device 2 by applying e.g. a digitalization, an amplification and a filtering in order to discern a stimulation pulse of the implantable pacemaker device 1 from intrinsic electrocardiogram signals originating from cardiac activity. For example, the processing circuitry 22 may process sensed electrocardiogram signals S2 by assessing a positive or negative flank of a pulse, for example by evaluating the flank's gradient or the flank' height, or by applying pattern recognition techniques in order to identify a predefined pulse shape stemming from a stimulation pulse as emitted by the pacemaker device 1.

In one embodiment, the processing circuitry 22 comprises a circular buffer 220 which serves to circularly store sensed electrocardiogram signals S2 such that a portion of the sensed electrocardiogram signals S2 relating to a prior time span is always kept in memory and is circularly overwritten by new data. For example, the circular buffer 220 may be configured to keep a signal portion for a time span between 10 seconds and 5 minutes, for example between 20 seconds and 1 minute, in memory such that always a most recent signal portion is stored within the circular buffer 220.

The processing circuitry 22 is configured to derive information from the sensed electrocardiogram signals S2 in relation to the operation of the pacemaker device 1. The processing circuitry 22 hence is operative to enable a monitoring of the operation of the pacemaker device 1 based solely on the reception of electrocardiogram signals S2 and by assessing the action of the pacemaker device 1 according to sensed electrocardiogram signals S2. Based on the monitoring of the action of the pacemaker device 1, data may be transmitted to the external device 3 in order to provide for a monitoring of the operation of the pacemaker device 1.

Namely, in one embodiment the processing circuitry 22 is configured to cause a transmission of information to the external device 3 based on the identification of one or multiple stimulation events Vp within the sensed electrocardiogram signal S2. For example, in case a stimulation event Vp caused by the pacemaker device 1 is identified within the electrocardiogram signal S2, a signal portion containing the stimulation event Vp may be transmitted within a data message to the external device 3, such that the external device 3 obtains information about the stimulation event Vp as caused by the pacemaker device 1.

In another example, the processing circuitry 22 may derive statistical information based on stimulation events Vp and may transmit the statistical information to the external device 3. Statistical information may include a frequency of stimulation, an average period between stimulation events, information concerning a ratio of intrinsic versus stimulated activity, statistical information relating to a cardiac response, and other statistical data.

In yet another example, the processing circuitry 22 may have information about a programmed function of the pacemaker device 1 stored in its memory such that the implantable medical device 2 comprises information about an operative setup of the pacemaker device 1. The implantable medical device 2 hence is enabled to assess the operation of the pacemaker device 1, based on sensed electrocardiogram signals S2, versus an expected operation. If the pacemaker device 1 functions according to its expected operation, this may be protocolled. Likewise, if the operation of the pacemaker device 1 deviates from its expected functioning, this may be monitored and for example warning messages may be triggered.

For example, the pacemaker device 1 may be configured to provide for an anti-tachycardia stimulation in case the implantable pacemaker device 1 detects a tachycardia based on its sensed signals as received via the electrode arrangement 11. In case a tachycardia is detected, anti-tachycardia stimulation pulses shall be emitted. This operation by the pacemaker device 1 may be monitored by the implantable medical device 2, which in its sensed electrocardiogram signals S2 may likewise identify a tachycardia and, subsequent to the identification of a tachycardia, may monitor whether the pacemaker device 1 correctly emits an anti-tachycardia stimulation signal. Signal portions relating to the operation of the pacemaker device 1 may be stored, in case the pacemaker device 1 correctly emits an anti-tachycardia stimulation signal as well as in case the pacemaker device 1 does not emit an anti-tachycardia stimulation signal contrary to its expected behavior.

In another example, the pacemaker device 1 may be configured to provide for an anti-bradycardia stimulation in case a bradycardia is detected by the pacemaker device 1. The operation of the pacemaker device 1 is monitored by the implantable medical device 2 by identifying a bradycardia in the sensed electrocardiogram signal S2 and by, subsequently, monitoring whether the pacemaker device 1 correctly emits an anti-bradycardia pacing in accordance with the detected bradycardia.

Based on a monitoring of stimulation events Vp in the sensed electrocardiogram signals S2 as caused by the pacemaker device 1, the implantable medical device 2 may cause a data communication with the external device 3.

In an event-based communication, for example, the implantable medical device 2 may for example trigger a communication message on the event of detecting a stimulation event Vp as caused by the pacemaker device 1 or on the event of detecting a deviation within the operation of the pacemaker device 1 from an expected operation. Based on the detection of a certain event, hence, a data message is created and transmitted to the external device 3 using the communication circuitry 24.

In addition or alternatively, information concerning the pacemaker device 1 may be included in data messages as transmitted to the external device 3 according to a regular, predefined communication scheme, within which for example data messages are exchanged in between the implantable medical device 2 and the external device 3 in a regular, prescheduled manner. The identification of a stimulation event Vp or the identification of a deviation from an expected operation of the pacemaker device 1 thus does not cause a triggering of a specific, dedicated communication message, but information relating to the operation of the pacemaker device 1 is included in a regular communication message, for example to provide for a periodic reporting.

The data transmission may contain portions of the sensed electrocardiogram signals S2 relating to e.g. an identified stimulation event Vp. In addition or alternatively, the data transmission may include information derived in connection with a stimulation event Vp, for example statistical information or timing information.

The idea of the invention is not limited to the embodiments described above, but may be implemented in an entirely different fashion.

The implantable medical device may in particular be an implantable cardioverter defibrillator device (ICD). In another embodiment, the implantable medical device may be a monitoring device, such as a so-called biomonitor, to be implanted e.g. subcutaneously in proximity to the heart for sensing and monitoring signals, which however does not by itself provide for a therapeutic function by emitting stimulation signals.

### List of reference numerals

- 1: Pacemaker device
- 10: Housing
- 11: Electrode arrangement
- 110, 111: Electrode poles
- 12: Control circuitry
- 13: Energy storage (battery)
- 2: Non-transvenous defibrillator device
- 20: Generator
- 21: Lead
- 211, 212: Sensing electrode
- 213: Shock electrode
- 22: Processing circuitry
- 220: Circular buffer
- 23: Energy storage (battery)
- 24: Communication circuitry
- 3: External device
- H: Heart
- L: Communication link
- P: Patient
- S1: Stimulation signal
- S2: Sensed signal
- Vp: Stimulation event

## Claims

1. An implantable medical device (2) for providing a diagnostic and/or therapeutic cardiac function within a patient (P), comprising:
a sensing arrangement for sensing electrocardiogram signals;
a communication circuitry (24) for data communication with a device (3) external to the patient (P); and
a processing circuitry (22) for processing sensed electrocardiogram signals (S2), wherein the processing circuitry (22) is configured to identify, based on the sensed electrocardiogram signals (S2), a stimulation event (Vp) caused by an implantable pacemaker device (1) and to cause a transmission of information concerning said identification of a stimulation event (Vp) to the device (3) external to the patient (P).

2. The implantable medical device (2) according to claim 1, **characterized in that** the implantable medical device (2) is an implantable non-transvenous defibrillator device configured to emit a shock pulse for achieving a defibrillation.

3. The implantable medical device (2) according to claim 2, **characterized in that** the implantable non-transvenous defibrillator device comprises a lead (21) to be implanted extracardially and a shock electrode (213) arranged on the lead (21).

4. The implantable medical device (2) according to one of claims 1 to 3, **characterized in that** the processing circuitry (22) is configured to store a portion of the sensed electrocardiogram signals (S2) dependent on said identification of a stimulation event (Vp) and to transmit a stored portion of the sensed electrocardiogram signals (S2) to the device (3) external to the patient (P).

5. The implantable medical device (2) according to claim 4, **characterized in that** the processing circuitry (22) comprises a circular buffer (220) for circularly storing the sensed electrocardiogram signals (S2).

6. The implantable medical device (2) according to claim 4 or 5, **characterized in that** said portion comprises a signal portion prior to the stimulation event (Vp), a signal portion containing the stimulation event (Vp) and/or a signal portion after the stimulation event (Vp).

7. The implantable medical device (2) according to one of the preceding claims, **characterized in that** the processing circuitry (22) is configured to statistically analyze a multiplicity of stimulation events (Vp) caused by the implantable pacemaker device (1) and to communicate statistical information concerning said multiplicity of stimulation events (Vp) to the device (3) external to the patient (P).

8. The implantable medical device (2) according to one of the preceding claims, **characterized in that** the processing circuitry (22) is configured to store programming information indicative of a programmed stimulation function of the implantable pacemaker device (1), wherein the processing circuitry (22) is configured to monitor said programmed stimulation function based on said identification of a stimulation event (Vp).

9. The implantable medical device (2) according to one of the preceding claims, **characterized in that** the processing circuitry (22) is configured to identify, based on the sensed electrocardiogram signals (S2), a requirement for a stimulation event (Vp) and to monitor, based on the sensed electrocardiogram signals (S2), whether the implantable pacemaker device (1) causes a stimulation event (Vp) in relation to an identification of a requirement for a stimulation event (Vp).

10. The implantable medical device (2) according to one of the preceding claims, **characterized in that** the processing circuitry (22) is configured to identify an anti-tachycardia stimulation event caused by the implantable pacemaker device (1) and to cause a transmission of information concerning said anti-tachycardia stimulation event to the device (3) external to the patient (P).

11. The implantable medical device (2) according to one of the preceding claims, **characterized in that** the processing circuitry (22) is configured to identify a requirement for an anti-bradycardia stimulation and to monitor whether the implantable pacemaker device (1) causes an anti-bradycardia stimulation event in relation to an identification of a requirement for an anti-bradycardia stimulation.

12. The implantable medical device (2) according to one of the preceding claims, **characterized in that** the processing circuitry (22) is configured to trigger a data communication message to the device (3) external to the patient (P) based on said identification of a stimulation event (Vp), or to include said information concerning said identification of a stimulation event (Vp) in a data message scheduled according to a pre-defined regular communication scheme.

13. An implantable therapy system, comprising: an implantable pacemaker device (1) for emitting a cardiac stimulation signal for achieving an intra-cardiac pacing; and an implantable medical device (2) according to one of the preceding claims.

14. The implantable therapy system according to claim 13, wherein the implantable pacemaker device (1) is a leadless pacemaker device.

## Patentansprüche

1. Implantierbare medizinische Vorrichtung (2) zum Bereitstellen einer diagnostischen und/oder therapeutischen Herzfunktion in einem Patienten (P), umfassend:
eine Erfassungsanordnung zum Erfassen von Elektrokardiogrammsignalen
eine Kommunikationsschaltung (24) zur Datenkommunikation mit einer Vorrichtung (3), die sich außerhalb des Patienten (P) befindet; und
eine Verarbeitungsschaltung (22) zum Verarbeiten erfasster Elektrokardiogrammsignale (S2), wobei die Verarbeitungsschaltung (22) konfiguriert ist, um ein Stimulationsereignis (Vp), das durch eine implantierbare Schrittmachervorrichtung (1) veranlasst wird, basierend auf den erfassten Elekrokardiogrammsignalen (S2) zu identifizieren und eine Übertragung von Information bezüglich der Identifizierung eines Stimulationsereignisses (Vp) an die Vorrichtung (3), die sich außerhalb des Patienten (P) befindet, zu veranlassen.

2. Implantierbare medizinische Vorrichtung (2) nach Anspruch 1, **dadurch gekennzeichnet, dass** es sich bei der implantierbaren medizinischen Vorrichtung (2) um eine implantierbare nicht-transvenöse Defibrillatorvorrichtung handelt, die konfiguriert ist, um einen Schockimpuls zum Erreichen einer Defibrillation abzugeben.

3. Implantierbare medizinische Vorrichtung (2) nach Anspruch 2, **dadurch gekennzeichnet, dass** die implantierbare nicht-transvenöse Defibrillatorvorrichtung eine extrakardial zu implantierende Zuleitung (21) und eine an der Zuleitung (21) angeordnete Schockelektrode (213) umfasst.

4. Implantierbare medizinische Vorrichtung (2) nach Anspruch 1 bis 3, **dadurch gekennzeichnet, dass** die Verarbeitungsschaltung (22) konfiguriert ist, um einen Abschnitt der erfassten Elektrokardiogrammsignale (S2) in Abhängigkeit von der Identifizierung eines Stimulationsereignisses (Vp) zu speichern und um einen gespeicherten Abschnitt der erfassten Elektrokardiogrammsignale (S2) an die Vorrichtung (3), die sich außerhalb des Patienten (P) befindet, zu übertragen.

5. Implantierbare medizinische Vorrichtung (2) nach Anspruch 4, **dadurch gekennzeichnet, dass** die Verarbeitungsschaltung (22) einen Ringpuffer (220) zum zirkulären Speichern der erfassten Elektrokardiogrammsignale (S2) umfasst.

6. Implantierbare medizinische Vorrichtung (2) nach Anspruch 4 oder 5, **dadurch gekennzeichnet, dass** der Abschnitt einen Signalabschnitt vor dem Stimulationsereignis (Vp), einen Signalabschnitt, der das Stimulationsereignis (Vp) enthält und/oder einen Signalabschnitt nach dem Stimulationsereignis (Vp) umfasst.

7. Implantierbare medizinische Vorrichtung (2) nach einem der vorhergehenden Ansprüche, **dadurch gekennzeichnet, dass** die Verarbeitungsschaltung (22) konfiguriert ist, um eine Vielzahl von Stimulationsereignissen (Vp), die durch die implantierbare Schrittmachervorrichtung (1) veranlasst werden, statistisch zu analysieren und um statistische Information bezüglich einer Vielzahl von Stimulationsereignissen (Vp) an die Vorrichtung (3), die sich außerhalb des Patienten (P) befindet, zu übermitteln.

8. Implantierbare medizinische Vorrichtung (2) nach einem der vorhergehenden Ansprüche, **dadurch gekennzeichnet, dass** die Verarbeitungsschaltung (22) konfiguriert ist, um Programmierinformation zu speichern, die auf eine programmierte Stimulationsfunktion der implantierbaren Schrittmachervorrichtung (1) hinweist, wobei die Verarbeitungsschaltung (22) konfiguriert ist, um die programmierte Stimulationsfunktion basierend auf der Identifizierung eines Stimulationsereignisses (Vp) zu überwachen.

9. Implantierbare medizinische Vorrichtung (2) nach einem der vorhergehenden Ansprüche, **dadurch gekennzeichnet, dass** die Verarbeitungsschaltung (22) konfiguriert ist, um eine Voraussetzung für ein Stimulationsereignis (Vp) basierend auf den erfassten Elektrokardiogrammsignalen (S2) zu identifizieren, und um basierend auf den erfassten Elektrokardiogrammsignalen (S2) zu überwachen, ob die implantierbare Schrittmachervorrichtung (1) ein Stimulationsereignis (Vp) in Verbindung mit einer Identifizierung einer Voraussetzung für ein Stimulationsereignis (Vp) veranlasst.

10. Implantierbare medizinische Vorrichtung (2) nach einem der vorhergehenden Ansprüche, **dadurch gekennzeichnet, dass** die Verarbeitungsschaltung (22) konfiguriert ist, um ein antitachykardes Stimulationsereignis zu identifizieren, das durch die implantierbare Schrittmachervorrichtung (1) veranlasst wird, und um eine Übertragung von Information bezüglich des antitachykarden Stimulationsereignisses an die Vorrichtung (3), die sich außerhalb des Patienten (P) befindet, zu veranlassen.

11. Implantierbare medizinische Vorrichtung (2) nach einem der vorhergehenden Ansprüche, **dadurch gekennzeichnet, dass** die Verarbeitungsschaltung (22) konfiguriert ist, um eine Voraussetzung für eine antibradykarde Stimulation zu identifizieren und zu überwachen, ob die implantierbare Schrittmachervorrichtung (1) ein antibradykardes Stimulationsereignis in Verbindung mit einer Identifizierung einer Voraussetzung für eine antibradykarde Stimulation veranlasst.

12. Implantierbare medizinische Vorrichtung (2) nach einem der vorhergehenden Ansprüche, **dadurch gekennzeichnet, dass** die Verarbeitungsschaltung (22) konfiguriert ist, um eine Datenkommunikationsnachricht an die Vorrichtung (3), die sich außerhalb des Patienten (P) befindet, basierend auf der Identifizierung eines Stimulationsereignisses (Vp) auszulösen, oder um die Information bezüglich der Identifizierung eines Stimulationsereignisses (Vp) in einer Datennachricht, die gemäß einem vordefinierten regelmäßigen Kommunikationsschema geplant ist, einzuschließen.

13. Implantierbares Therapiesystem, umfassend: eine implantierbare Schrittmachervorrichtung (1) zum Abgeben eines Herzstimulationssignals zum Erreichen einer intrakardialen Stimulation; und eine implantierbare medizinische Vorrichtung (2) nach einem der vorhergehenden Ansprüche.

14. Implantierbares Therapiesystem nach Anspruch 13, wobei es sich bei der implantierbaren Schrittmachervorrichtung (1) um eine kabellose Schrittmachervorrichtung handelt.

## Revendications

1. Dispositif médical implantable (2) destiné à fournir une fonction cardiaque diagnostique et/ou thérapeutique au sein d'un patient (P), comprenant :
un agencement de détection destiné à détecter des signaux d'électrocardiogramme ;
un circuit de communication (24) destiné à la communication de données avec un dispositif (3) externe au patient (P) ; et
un circuit de traitement (22) destiné au traitement de signaux d'électrocardiogramme (S2) détectés, dans lequel le circuit de traitement (22) est configuré pour identifier, sur la base des signaux d'électrocardiogramme (S2) détectés, un événement de stimulation (Vp) amené par un dispositif de stimulateur cardiaque implantable (1) et pour amener une transmission d'informations concernant ladite identification d'un événement de stimulation (Vp) au dispositif (3) externe au patient (P).

2. Dispositif médical implantable (2) selon la revendication 1, **caractérisé en ce que** le dispositif médical implantable (2) est un dispositif de défibrillateur non transveineux implantable configuré pour émettre une impulsion de choc afin d'obtenir une défibrillation.

3. Dispositif médical implantable (2) selon la revendication 2, **caractérisé en ce que** le dispositif de défibrillateur non transveineux implantable comprend un câble (21) à implanter de manière extracardiaque et une électrode de choc (213) agencée sur le câble (21).

4. Dispositif médical implantable (2) selon l'une des revendications 1 à 3, **caractérisé en ce que** le circuit de traitement (22) est configuré pour stocker une partie des signaux d'électrocardiogramme (S2) détectés en fonction de ladite identification d'un événement de stimulation (Vp) et pour transmettre une partie stockée des signaux d'électrocardiogramme (S2) détectés au dispositif (3) externe au patient (P).

5. Dispositif médical implantable (2) selon la revendication 4, **caractérisé en ce que** le circuit de traitement (22) comprend un tampon circulaire (220) pour stocker de manière circulaire les signaux d'électrocardiogramme (S2) détectés.

6. Dispositif médical implantable (2) selon la revendication 4 ou 5, **caractérisé en ce que** ladite partie comprend une partie de signal avant l'événement de stimulation (Vp), une partie de signal contenant l'événement de stimulation (Vp) et/ou une partie de signal après l'événement de stimulation (Vp).

7. Dispositif médical implantable (2) selon l'une des revendications précédentes, **caractérisé en ce que** le circuit de traitement (22) est configuré pour analyser de manière statistique une multiplicité d'événements de stimulation (Vp) amenés par le dispositif de stimulateur cardiaque implantable (1) et pour communiquer des informations statistiques concernant ladite multiplicité d'événements de stimulation (Vp) au dispositif (3) externe au patient (P).

8. Dispositif médical implantable (2) selon l'une des revendications précédentes, **caractérisé en ce que** le circuit de traitement (22) est configuré pour stocker des informations de programmation indiquant une fonction de stimulation programmée du dispositif de stimulateur cardiaque implantable (1), dans lequel le circuit de traitement (22) est configuré pour surveiller ladite fonction de stimulation programmée en se basant sur ladite identification d'un événement de stimulation (Vp).

9. Dispositif médical implantable (2) selon l'une des revendications précédentes, **caractérisé en ce que** le circuit de traitement (22) est configuré pour identifier, en se basant sur les signaux d'électrocardiogramme (S2) détectés, une exigence pour un événement de stimulation (Vp) et pour surveiller, en se basant sur les signaux d'électrocardiogramme (S2) détectés, si le dispositif de stimulateur cardiaque implantable (1) amène un événement de stimulation (Vp) en lien avec une identification d'une exigence pour un événement de stimulation (Vp).

10. Dispositif médical implantable (2) selon l'une des revendications précédentes, **caractérisé en ce que** le circuit de traitement (22) est configuré pour identifier un événement de stimulation anti-tachycardie amené par le dispositif de stimulateur cardiaque implantable (1) et pour amener une transmission d'informations concernant ledit événement de stimulation anti-tachycardie au dispositif (3) externe au patient (P).

11. Dispositif médical implantable (2) selon l'une des revendications précédentes, **caractérisé en ce que** le circuit de traitement (22) est configuré pour identifier une exigence pour une stimulation anti-bradycardie et pour surveiller si le dispositif de stimulateur cardiaque implantable (1) amène un événement de stimulation anti-bradycardie en lien avec une identification d'une exigence pour une stimulation anti-bradycardie.

12. Dispositif médical implantable (2) selon l'une des revendications précédentes, **caractérisé en ce que** le circuit de traitement (22) est configuré pour déclencher un message de communication de données au dispositif (3) externe au patient (P) en se basant sur ladite identification d'un événement de stimulation (Vp), ou pour inclure lesdites informations concernant ladite identification d'un événement de stimulation (Vp) dans un message de données planifié conformément à un schéma de communication régulier prédéfini.

13. Système thérapeutique implantable, comprenant : un dispositif de stimulateur cardiaque implantable (1) destiné à émettre un signal de stimulation cardiaque pour obtenir une stimulation intracardiaque ; et un dispositif médical implantable (2) selon l'une des revendications précédentes.

14. Système thérapeutique implantable selon la revendication 13, dans lequel le dispositif de stimulateur cardiaque implantable (1) est un dispositif de stimulateur cardiaque sans câbles.
